# EUROPÄISCHE PATENTANMELDUNG

(11) **EP 4 684 748 A1**
(43) Veröffentlichungstag der Anmeldung: **28.01.2026**
(21) Anmeldenummer: 24190249.3
(22) Anmeldetag: 23.07.2024
(51) Int. Cl.: A61B 18/12, A61N 1/40, A61B 18/00, A61N 1/06

(54) **VORRICHTUNG UND VERFAHREN ZUR ERZEUGUNG VON HOCHFREQUENTEN STRÖMEN ZUR BEHANDLUNG VON GEWEBE**

(71) Anmelder: Wellcomet GmbH, 76646 Bruchsal (DE)
(72) Erfinder: KRUGLIKOV, Ilja, 76351 Linkenheim-Hochstetten (DE)
(74) Vertreter: LBP Lemcke, Brommer & Partner Patentanwälte mbB

(57) **Zusammenfassung**

Vorgeschlagen wird eine Vorrichtung zur Erzeugung von hochfrequenten Strömen, zur kosmetischen und/oder therapeutischen Behandlung von Gewebe mittels Elektroden der Vorrichtung, umfassend:
eine Hochfrequenz-Energiequelle zur Erzeugung von hochfrequenten Strömen und mindestens ein erstes Elektrodenpaar mit zwei Elektroden zum Beaufschlagen des Gewebes mit den hochfrequenten Strömen, welche Elektroden mit jeweils einem Ausgang der Hochfrequenz-Energiequelle verbunden sind,
welche Vorrichtung ausgebildet ist, mittels der Hochfrequenz-Energiequelle die hochfrequenten Ströme mit mehreren unterschiedlichen Frequenzen fⱼ zu erzeugen,
dadurch gekennzeichnet, dass
die Vorrichtung ausgebildet ist, mittels der Hochfrequenz-Energiequelle die Ströme mit mehreren unterschiedlichen Frequenzen fⱼ in einer zeitlichen Sequenz mit Sequenzschritten Sⱼ mit j=1, 2, ..., M; M E N, zu erzeugen,
jedem Sequenzschritt Sⱼ zumindest ein in diesem Sequenzschritt Sⱼ erzeugter Strom mit der Frequenz fⱼ und eine Zeitdauer Tⱼ, für welche der Strom mit der Frequenz fⱼ in dem Sequenzschritt Sⱼ erzeugt wird, zugeordnet ist, und
zumindest eine Abfolge der Frequenzen fⱼ eine zufällige Abfolge ist.

Außerdem ist wenigstens eines der folgenden weiteren Merkmale realisiert: Die Stromstärke der Ströme liegt zwischen 100 mA und 2 A, vorzugsweise zwischen 300 mA und 1 A. Die Frequenzen fⱼ liegen in einem Frequenzbereich zwischen 0,1 MHz und 20 MHz, vorzugsweise zwischen 0,1 MHz und 10 MHz, höchst vorzugsweise zwischen 0,1 MHz und 3 MHz. Die Zeitdauer Tⱼ, ist kleiner 100 ms, vorzugsweise kleiner 50 ms.

Außerdem vorgeschlagen wird ein entsprechendes, insbesondere kosmetisches Behandlungserfahren.

## Beschreibung

Die Erfindung betrifft eine Vorrichtung nach dem Oberbegriff des Anspruchs 1 zur Erzeugung von hochfrequenten Strömen zur kosmetischen und/oder therapeutischen Behandlung von Gewebe mittels Elektroden der Vorrichtung. Eine solche Vorrichtung umfasst eine Hochfrequenz-Energiequelle zur Erzeugung von hochfrequenten Strömen und mindestens ein erstes Elektrodenpaar mit zwei Elektroden zum Beaufschlagen des Gewebes mit den hochfrequenten Strömen, welche Elektroden mit jeweils einem Ausgang der Hochfrequenz-Energiequelle verbunden sind. Die Vorrichtung ist dazu ausgebildet, mittels der Hochfrequenz-Energiequelle die hochfrequenten Ströme mit mehreren unterschiedlichen Frequenzen fⱼ zu erzeugen.

Die Erfindung betrifft auch ein Verfahren nach dem Oberbegriff des Anspruchs 13 zur insbesondere kosmetischen Behandlung von Gewebe mittels hochfrequenten Strömen, mit den Verfahrensschritten: Anordnen zumindest eines Elektrodenpaars an dem Gewebe und Beaufschlagen des Gewebes mittels des Elektrodenpaares mit hochfrequentem Strom.

Derartige Vorrichtungen und Verfahren sind bekannt, insbesondere aus den Schutzrechten DE 10 2016 120 138 B4, EP 2 022 429 B1 und EP 3 097 881 B1 der Anmelderin.

Solche Vorrichtungen bzw. Verfahren finden Anwendung in der Thermotherapie von insbesondere menschlichem Gewebe. Grundprinzip ist hierbei die Erwärmung des Gewebes und hierdurch die Erzeugung von reversiblen oder irreversiblen Veränderungen. Typische Anwendungsgebiete sind beispielsweise die thermische Behandlung von instabilen Gelenkkapseln mittels thermischer Kapsulorrhaphie ("Thermal Collagen Shrinkage"), Thermokeratoplastie, Skin Resurfacing sowie die Hautfestigung ("Skin Tightening").

All diesen Anwendungen ist gemeinsam, dass mittels Wärme Kollagennetzwerkstrukturen verändert werden. Die bekanntesten Erwärmungsmethoden für Kollagengewebe sind die Lasererwärmung, sowie die Erwärmung mit hochfrequenten Strömen, welche auch als "radiofrequente Ströme" oder RF-Ströme bezeichnet werden.

Das Grundprinzip bei der Erwärmung mittels hochfrequenter Ströme, wie es zumindest teilweise auch der vorliegenden Erfindung zugrunde liegt, ist das Einbringen von hochfrequenten Strömen mittels Elektroden in das Gewebe. Typische Vorrichtungen hierzu weisen eine Hochfrequenz-Energiequelle zur Erzeugung der hochfrequenten Ströme auf, sowie wenigstens ein Elektrodenpaar. Die Elektroden des Elektrodenpaares sind mit der Hochfrequenz-Energiequelle verbunden und weisen jeweils eine Kontaktfläche auf, mittels derer sie an das zu beaufschlagende Gewebe angelegt werden. Über die Kontaktflächen wird das Gewebe mit den von der Hochfrequenz-Energiequelle erzeugten Strömen beaufschlagt, sodass aufgrund des Stromflusses durch Joulesche Wärme eine Erwärmung des Gewebes stattfindet.

Wesentlich bei solchen Behandlungen ist, dass einerseits eine Schmerzschwelle des Patienten nicht überschritten werden soll und andererseits eine Begrenzung aufgrund der Thermotoleranzgrenze für Gewebeschädigung gegeben ist. Diese Probleme treten insbesondere an den Kontaktflächen der Elektroden mit dem Gewebe auf.

In der Vergangenheit hat man u.a. versucht, dieser Problematik durch Anpassung der Leistung des verwendeten Stroms und/oder Anpassen der Beaufschlagungsdauer zu begegnen (sog. "High Rate"-Anwendung mit relativ hoher Leistung und nur einmaliger Beaufschlagung oder sog. "Low Rate"-Anwendungen, bei den eine niedrigere Leistung über einen längeren Zeitraum eingesetzt wird).

Aus einschlägigen Forschungsarbeiten des Erfinders ist bekannt, dass speziell bei der Behandlung von Indikationen mit starker Veränderung der Hautstruktur ein besonderes Augenmerk auf Grenzflächen innerhalb der Haut gerichtet werden sollte, die sog. "weak links" also Schwachstellen darstellen, vgl. Ilja L. Kruglikov, "Assessment of Mechanical Stress Induced by Radiofrequency Currents on Skin Interfaces", Hindawi, 2021, Volume 2021, 2021 (1): 6623757.

In dem Zusammenhang wurde seitens des Erfinders erkannt, dass allein mit einer Temperatureinwirkung (sog. Hyperthermie) diese Grenzflächen, z.B. zwischen Epidermis und Dermis oder zwischen Dermis und Subkutis, nicht selektiv beeinflusst werden können, was jedoch wesentlich für einen guten Behandlungserfolg ist.

Außerdem besteht allgemein bei der Behandlung von Gewebe mit Strom die Gefahr, dass sich das Gewebe an die Wirkung des Stroms gewöhnt, wenn die Behandlungsdauer zu lang ist, was den Behandlungserfolg weiter schmälern kann.

Der vorliegenden Erfindung liegt die Aufgabe zugrunde, die bekannte Vorrichtung zur Erzeugung von hochfrequenten Strömen und das oben definierte Verfahren dahingehend weiter zu verbessern, dass ohne eine wesentliche Erhöhung der thermischen Gewebeoberflächenbelastung an den Kontaktstellen mit den Elektroden eine Erhöhung der in das Gewebe einführbaren Energie und damit der im Gewebe erzeugten Wärme ermöglicht wird. Weiterhin soll die Erhöhung der Wärmeerzeugung erfolgen, ohne die Schmerzschwelle des Patienten einerseits oder eine Thermotoleranzgrenze für Gewebeschädigung an den Kontaktstellen andererseits zu überschreiten. Dabei soll die insgesamt zugeführte Leistung nach Möglichkeit erhöht werden, ohne dass es zu Schädigungen oder Gewöhnungseffekten kommt, welche die Effizienz der Behandlung beeinträchtigen können. Des Weiteren soll gezielt die Beeinflussung der o.g. "weak links" in Form von Grenzflächen der Gewebestruktur ermöglicht werden.

Gelöst ist diese Aufgabe durch eine Vorrichtung zur Erzeugung von hochfrequenten Strömen, zur kosmetischen und/oder therapeutischen Behandlung von Gewebe mittels Elektroden der Vorrichtung, gemäß Anspruch 1 und durch ein Verfahren zur Behandlung von Gewebe, insbesondere zur kosmetischen Behandlung von Gewebe, mittels hochfrequenten Strömen gemäß Anspruch 13.

Vorteilhafte Ausgestaltungen der erfindungsgemäßen Vorrichtung und des erfindungsgemäßen Verfahrens finden sich in den abhängigen Ansprüchen.

Eine erfindungsgemäße Vorrichtung zur Erzeugung von hochfrequenten Strömen, zur kosmetischen und/oder therapeutischen Behandlung von Gewebe mittels Elektroden der Vorrichtung, umfasst eine Hochfrequenz-Energiequelle zur Erzeugung von hochfrequenten Strömen und mindestens ein erstes Elektrodenpaar mit zwei Elektroden zum Beaufschlagen des Gewebes mit den hochfrequenten Strömen, welche Elektroden mit jeweils einem Ausgang der Hochfrequenz-Energiequelle verbunden sind. Die Vorrichtung ist dazu ausgebildet, mittels der Hochfrequenz-Energiequelle die hochfrequenten Ströme mit mehreren unterschiedlichen Frequenzen fⱼ in einer zeitlichen Sequenz mit Sequenzschritten Sⱼ mit j=1, 2, ..., M; M E N, zu erzeugen, wobei jedem Sequenzschritt Sⱼ zumindest ein in diesem Sequenzschritt Sⱼ erzeugter Strom mit der Frequenz fⱼ und eine Zeitdauer Tⱼ, für welche der Strom mit der Frequenz fⱼ in dem Sequenzschritt Sⱼ erzeugt wird, zugeordnet ist, und wobei zumindest eine Abfolge der Frequenzen fⱼ eine zufällige Abfolge ist. Die Vorrichtung ist zusätzlich ausgebildet, die Ströme mit einer Stromstärke zwischen 100 mA und 2 A, vorzugsweise zwischen 300 mA und 1 A zu erzeugen, und/oder die Ströme in einem Frequenzbereich zwischen 0,1 MHz und 20 MHz, vorzugsweise zwischen 0,1 MHz und 10 MHz, höchst vorzugsweise zwischen 0,1 MHz und 3 MHz, zu erzeugen, und/oder die Ströme während einer Zeitdauer Tⱼ, kleiner 100 ms, vorzugsweise kleiner 50 ms, zu erzeugen.

Ein erfindungsgemäßes Verfahren zur insbesondere kosmetischen Behandlung von Gewebe mittels hochfrequenten Strömen beinhaltet die Verfahrensschritte des Anordnens zumindest eines Elektrodenpaars an dem Gewebe und des Beaufschlagens des Gewebes mittels des Elektrodenpaares mit hochfrequentem Strom. Das Verfahren zeichnet sich dadurch aus, dass das Gewebe sequentiell mit Strömen mit mehreren unterschiedlichen Frequenzen fⱼ beaufschlagt wird, die in einer zeitlichen Sequenz mit Sequenzschritten Sⱼ mit j=1, 2, ..., M; M E N, aufeinander folgen, wobei in jedem Sequenzschritt Sⱼ das Gewebe mit Strom einer diesem Sequenzschritt zugeordneten Frequenz fⱼ während einer Zeitdauer Tⱼ, beaufschlagt wird, und zumindest die Frequenzen fⱼ zufällig aufeinander folgen, während zusätzlich wenigstens eines der folgenden weiteren Merkmale realisiert wird: 1) eine Stromstärke der Ströme wird zwischen 100 mA und 2 A, vorzugsweise zwischen 300 mA und 1 A, gewählt; und/oder 2) das Gewebe wird mit Strömen mit einer Frequenz fⱼ in einem Frequenzbereich zwischen 0,1 MHz und 20 MHz, vorzugsweise zwischen 0,1 MHz und 10 MHz, höchst vorzugsweise zwischen 0,1 MHz und 3 MHz, beaufschlagt; und/oder 3) die Zeitdauer Tⱼ, beträgt weniger als 100 ms, vorzugsweise weniger als 50 ms.

Das Verfahren ist explizit nicht auf eine nur kosmetische Behandlung von Gewebe beschränkt, sondern kann bei allen Indikationen zur Anwendung kommen, die mit einer starken Veränderung an Grenzflächen der Haut verbunden sind. Beispiele für kosmetische Beghandlungen sind Hautalterung ("Skin Ageing") oder Cellulitis, Hautfestigung ("SkinTightening").

Die Sequenzschritte Sⱼ definieren demnach Zeitintervalle, während denen das Gewebe mit einem hochfrequenten Strom der Frequenz fⱼ beaufschlagt wird. Die Dauer der Beaufschlagung selbst ist Tⱼ, wobei Tⱼ das gesamte Zeitintervall des betreffenden Sequenzschritts Sⱼ abdecken kann oder auch nur einen Bruchteil desselben. Außerdem kann Tⱼ beliebig innerhalb des Zeitintervalls Sⱼ angeordnet sein, also z.B. genau mittig oder zu Beginn oder am Ende. Für jeden Sequenzschritt Sⱼ kann außerdem eine eigene zugehörige Zeit Tⱼ gewählt werden. Die Sequenzschritte Sⱼ können, müssen jedoch nicht zwingend gleich lang sein.

Dass die verwendeten Frequenzen fⱼ zufällig bzw. stochastisch aufeinander folgen, bedeutet im Rahmen der Erfindung, dass es keine Korrelation zwischen den in den einzelnen Sequenzschritten verwendeten Frequenzen gibt. Der Fachmann kennt verschiedenen Möglichkeiten oder Verfahren, derartige Zufälligkeiten zu erzeugen, die im Rahmen der Erfindung Verwendung finden können, z.B. mit Anwendung eines Zufallsgenerators, der diese Zahlen nach dem Muster eines weißen oder farbigen Rauschens erzeugt.

Konkret heißt das: in einem Sequenzschritt Sⱼ₋₁ wird das Gewebe während der Zeitdauer Tⱼ₋₁ mit Strom der Frequenz fⱼ₋₁ beaufschlagt, was auch als Puls bezeichnet wird; im folgenden Sequenzschritt Sⱼ wird das Gewebe während der Zeitdauer Tⱼ mit Strom der Frequenz fⱼ beaufschlagt; im folgenden Sequenzschritt Sⱼ₊₁ wird das Gewebe während der Zeitdauer Tⱼ₊₁ mit Strom der Frequenz fⱼ₊₁ beaufschlagt, usw. Die Zeitdauern der Beaufschlagung (also der Pulse) können dabei den Sequenzschritten längenmäßig im Wesentlichen, d.h. bis auf kurze Unterbrechungen beim Ändern der Frequenz entsprechen.

Typischerweise folgen eine bestimmte, endliche Anzahl von Pulsen aufeinander, bevor die Behandlung ab- bzw. unterbrochen und dann bei Bedarf bevorzugt an einem anderen Ort fortgesetzt wird.

Die Anmelderin hat erkannt, dass die im Anspruch 1 bzw. 13 enthaltenen Werte für Stromstärke, Frequenz und Pulslänge (Zeitdauer) besonders vorteilhaft für den angestrebten Behandlungserfolg sind. Dies gilt bereits für jeden der genannten Werte isoliert, vorzugsweise aber auch für die in den Ansprüchen enthaltene Kombination zweier oder sogar aller dieser Merkmale.

Insbesondere dann, wenn man speziell die selektive Beeinflussung von Gewebe-Grenzflächen (die genannten "weak links", siehe Kruglikov and Scherer, "Skin Aging as a mechanical phenomenon. The main weak links, Nutrition and healthy aging, 2018, 4(4), pp. 291-307) im Fokus hat, kann eine Frequenzwahl in dem angegebenen Bereich eine Beeinflussung des Gewebes sowohl durch Wärme (bei höheren Frequenzen) als auch durch Druck (bei niedrigeren Frequenzen) ermöglichen, und zwar gezielt an den genannten Grenzflächen. Die Wahl einer relativen kurzen Pulslänge (Zeitdauer) und die zufälligen Frequenzen bewirken, dass keine Gewöhnungseffekte auftreten. Die relativ hohe Stromstärke sorgt für einen ausreichenden Wirkungseintrag. Die verwendete Stromstärke ist deutlich höher als bei vorbekannten Vorrichtungen und wird durch die stochastische Frequenzauswahl begünstigt bzw. überhaupt erst ermöglicht, was den Behandlungserfolg verbessern kann.

Es wurde schon betont, dass jeder dieser Parameter - Stromstärke, Frequenz und Pulslänge (Zeitdauer) - für sich genommen bereits eine substantielle Verbesserung gegenüber dem Stand der Technik erreicht, dass jedoch insbesondere die Kombination aller drei Parameter die erreichbaren Vorteile in voller Stärke zur Entfaltung bringen kann. Vorteilhafter Weise bewirkt speziell die zufällige Frequenzabfolge bei entsprechender Frequenzwahl, dass neben weiter reduzierten Gewöhnungseffekten ein ständiger Wechsel insbesondere auch zwischen thermischer und druckbasierter Wirkung erfolgt, was den Behandlungserfolg weiter verbessert. Der Erfinder hat erkannt, dass bei einer Frequenz von etwa 300 kHz eine Grenze zwischen diesen beiden Regimes gezogen werden kann, siehe Kruglikov, a.a.O., Tabelle 4. Auf diese Weise sieht sich das behandelte Gewebe einer Art "biologischer Interferenz" ausgesetzt, bei der die unterschiedlichen Einwirkungen zwar physikalisch sequentiell, auf biologischen Zeitskalen jedoch quasi gleichzeitig stattfinden, was sich nach Erkenntnissen der Anmelderin sehr positiv auswirkt.

Die Hochfrequenz-Energiequelle kann in an sich bekannter Weise ausgebildet sein. Hochfrequent bedeutet im Rahmen der Erfindung, dass der Strom eine Frequenz zwischen 0,1 MHz und 20 MHz, vorzugsweise zwischen 0,1 MHz und 10 MHz, höchst vorzugsweise zwischen 0,1 MHz und 3 MHz, aufweist, wie oben bereits angegeben.

Die zu den Frequenzen fⱼ gehörigen Ströme können in ihrer jeweiligen Stromstärke (Amplitude Aⱼ) zwischen gewissen Grenzen fest oder frei (zufällig) gewählt werden, oder es kann eine Gewichtung erfolgen, insbesondere eine farbliche Gewichtung, wie sie von physikalischen Rauscheffekten bekannt ist.

Wie bereits angegeben, beträgt die minimale Stromstärke insbesondere 100 mA, bevorzugt 300 mA. Weiterhin beträgt die maximale Stromstärke insbesondere 2 A, bevorzugt 1 A.

Farbige oder farbliche Gewichtung bei der Erzeugung der Frequenzen bedeutet im Rahmen der Erfindung, dass die gewählten Frequenzen fⱼ - innerhalb der vorstehend genannten Grenzen - auf eine bestimmte Art verteilt sind. Analog zu weißem Rauschen kann insbesondere bevorzugt sein, dass die Frequenzen gleichmäßig zwischen den genannten Grenzen verteilt angeordnet sind. Im zeitlichen Mittel werden dann verschiedene gleich große spektrale Bereiche innerhalb der Grenzen gleich oft angesprochen. Insbesondere erfährt das Gewebe dann etwa gleich oft thermische und druckbasierte Beaufschlagung. Allerdings kann auch eine andere (farbliche) Gewichtung der Frequenzen erfolgen, allgemein mittels einer Gewichtungsfunktion g(f), um z.B. im zeitlichen Mittel gezielt mehr thermische oder mehr druckbasierte Beaufschlagung zu realisieren.

Die Anmelderin hat erkannt, dass durch die stochastische Auswahl der Frequenzen Gewöhnungseffekte des Gewebes vermieden werden, worauf weiter oben schon hingewiesen wurde. Außerdem ist es möglich, über längere Zeiten und/oder mit höheren Stromstärken zu arbeiten als im Stand der Technik, ohne dass es zu Schädigungen des Gewebes kommt. Es ist auch möglich, höhere Stromstärken zu verwenden, wodurch der Wirkeffekt verbessert werden kann.

Folgende Ausgestaltungen der Vorrichtung und des Verfahrens haben sich als besonders günstig erwiesen:
Eine Weiterbildung der erfindungsgemäßen Vorrichtung sieht vor, dass zusätzlich eine Abfolge der Zeitdauern Tⱼ, eine zufällige Abfolge ist.

Eine entsprechende Weiterbildung des erfindungsgemäßen Verfahrens sieht vor, dass zusätzlich die Zeitdauern Tⱼ, zufällig aufeinander folgen.

Hierauf wurde weiter oben schon hingewiesen. Die Zeitdauern Tⱼ, können von Sequenzschritt zu Sequenzschritt zufällig variieren. Das kann die Dauer der Strombeaufschlagung als solche betreffen und/oder die Lage des Beaufschlagungszeitraums (des Pulses) innerhalb des Sequenzschritts. Hierdurch kann das Gewebe geschont werden. Außerdem trägt dies zusätzlich zur Vermeidung von Gewöhnungseffekten bei.

Eine besonders bevorzugte Ausgestaltung der Vorrichtung sieht allerdings vor, dass eine zeitliche Länge der Sequenzschritte und die zugehörigen Zeitdauern Tⱼ, einander im Wesentlichen entsprechen, dass also jeder Sequenzschritt praktisch vollständig - bis auf etwaige technisch bedingte Unterbrechungen im Bereich von 1 ms oder kürzer - für die Strombeaufschlagung genutzt wird. Die Zeitdauern Tⱼ, sind vorzugsweise kürzer als 100 ms oder sogar kürzer als 50 ms. Dabei können die einzelnen Sequenzschritte gleich lang ausgebildet sein, oder sie können unterschiedliche Dauern aufweisen.

Eine Weiterbildung der erfindungsgemäßen Vorrichtung sieht vor, dass die Vorrichtung ausgebildet ist, mittels der Hochfrequenz-Energiequelle die Ströme mit mehreren unterschiedlichen Frequenzen fⱼ zu erzeugen, sodass die Frequenzen fⱼ gleichmäßig oder gemäß einer vorgegebenen Gewichtungsfunktion g(f) über den Frequenzbereich verteilt sind, worauf weiter oben schon hingewiesen wurde.

Besonders vorteilhaft ist hierbei, dass eine gezielte Gewichtung der verschiedenen Behandlungsregime (thermisch oder mechanischen Druck erzeugende) erreicht werden kann, je nach beabsichtigter Wirkung oder zu behandelndem Gewebe.

Entsprechende Ausgestaltungen des erfindungsgemäßen Verfahrens sehen vor, dass die Ströme mit mehreren unterschiedlichen Frequenzen fⱼ erzeugt werden, die gleichmäßig (analog zu weißem Rauschen) oder gemäß einer anderen vorgegebenen Gewichtungsfunktion g(f) über den Frequenzbereich verteilt sind.

Eine wieder andere Weiterbildung der erfindungsgemäßen Vorrichtung sieht vor, dass die Vorrichtung ausgebildet ist zum Erzeugen von langen Pulsen (auch als sog. Pulstrains bezeichnet) mit einer Dauer von mindestens 1 s, vorzugsweise 5 s, höchst vorzugsweise 10 s, während der das Gewebe an einem vorgegebenen, festen Ort mit den hochfrequenten Strömen beaufschlagt ist.

Derartige sog. "long pulses" bzw. "Pulsetrains" werden durch die erfindungsgemäße Ausgestaltung der Vorrichtung überhaupt erst ermöglicht, weil sie bei vorbekannten Vorrichtungen zu einer Gewebeschädigung führen würden. Derartige lange Pulse beinhalten also insbesondere nicht, dass die Zeitdauern der einzelnen Frequenzbeaufschlagungen erhöht würden, sondern stehen für die Dauer der Behandlung an einem vorgegebenen, festen Ort.

Eine entsprechende Weiterbildung des Verfahrens sieht vor, dass das Elektrodenpaar für eine Dauer von mindestens 1 s, vorzugsweise 5 s, höchst vorzugsweise 10 s, während der das Gewebe mit den hochfrequenten Strömen beaufschlagt wird, an einem festen Ort an dem Gewebe angeordnet wird bzw. bleibt. Dies kann automatisch oder manuell gesehen.

Noch eine andere Weiterbildung der erfindungsgemäßen Vorrichtung sieht vor, dass die Vorrichtung zumindest ein zweites Elektrodenpaar mit zwei Elektroden aufweist, die mit jeweils einem Ausgang der Hochfrequenz-Energiequelle verbunden sind. Außerdem ist die Hochfrequenz-Energiequelle dazu ausgebildet, die zwei Elektrodenpaare in wenigstens einem Sequenzschritt Sⱼ, vorzugsweise in im Wesentlichen allen Sequenzschritten Sⱼ, mit Strömen unterschiedlicher Frequenz fᵢ zu beaufschlagen.

Dadurch kann der Behandlungserfolg noch weiter verbessert werden. Es ist in diesem Kontext möglich, aber nicht erforderlich, dass beide Elektrodenpaare mit zufällig ausgewählten Frequenzen fᵢ zu beaufschlagt werden.

Eine besonders vorteilhafte Weiterbildung der erfindungsgemäßen Vorrichtung sieht vor, dass eines der Elektrodenpaare als monopolares Elektrodenpaar ausgebildet ist, das eine Flächenkontaktelektrode mit einer Gewebekontaktfläche vorzugsweise größer 5 cm² und eine Kleinkontaktelektrode mit einer Gewebekontaktfläche vorzugsweise kleiner 2 cm² umfasst. Das andere Elektrodenpaar ist als bipolares Elektrodenpaar ausgebildet, das zwei Kleinkontaktelektroden mit einer Gewebekontaktfläche von jeweils vorzugsweise kleiner 2 cm² umfasst.

Die Eigenschaften und Vorteile derartiger Elektroden sind aus der EP 2 022 429 B1 bekannt, deren Offenbarung durch Bezugnahme vollständig mit in die vorliegende Beschreibung aufgenommen wird. Die dort beschriebenen Vorteile können durch die zufällige Ansteuerung wenigstens eines Elektrodenpaares noch verbessert werden.

Allgemein hat sich die Verwendung von Elektroden mit einer Fläche zwischen etwa 50 mm² und etwa 2 cm² als sinnvoll und vorteilhaft erwiesen.

Eine wieder andere, besonders vorteilhafte Weiterbildung der erfindungsgemäßen Vorrichtung sieht vor, dass eines der Elektrodenpaare mit Strömen mit einer zufälligen Abfolge von Frequenzen fⱼ beaufschlagt ist, vorzugsweise das oben genannte monopolare Elektrodenpaar, während das andere Elektrodenpaar mit Strömen mit einer vorbestimmten Abfolge von Frequenzen fⱼ (oder mit einer festen Frequenz) beaufschlagt ist.

Durch die zufällige Beaufschlagung insbesondere des monopolaren Elektrodenpaares lassen sich Gesundheitsrisiken, die inhärent mit der Ausbildung einer Elektrode als relativ große Flächenkontaktelektrode verknüpft sind, sicher vermeiden, ohne auf den Einsatz derartiger Elektroden und/oder relativ starker Ströme verzichten zu müssen.

Eine bestimmte Weiterbildung dieser Ausgestaltung der erfindungsgemäßen Vorrichtung sieht vor, dass beide Elektrodenpaare mit Strömen mit einer zufälligen Abfolge von Frequenzen fⱼ beaufschlagt sind.

Auf diese Weise kommen die Vorteile des erfindungsgemäßen Ansatzes besonders gut zum Tragen.

In Weiterbildung der erfindungsgemäßen Vorrichtung kann noch vorgesehen sein, dass beide Elektrodenpaare alternierend mit Strömen mit einer zufälligen Abfolge von Frequenzen fⱼ beaufschlagt sind.

Dies bedeutet, dass ein Elektrodenpaar stochastisch beaufschlagt wird, während des andere Elektrodenpaar mit einer festen Frequenz (oder einer vorgegebenen Frequenzabfolge) beaufschlagt ist, und dass diese Beaufschlagung nach einer gewissen Zeit wechselt. Die Anmelderin hat hiermit besonders gute Behandlungsergebnisses erzielt.

In Weiterbildung der erfindungsgemäßen Vorrichtung kann auch noch vorgesehen sein, dass das erste Elektrodenpaar als bipolares Elektrodenpaar ausgebildet ist, und die Vorrichtung eine Mehrzahl weiterer bipolare Elektrodenpaare, insbesondere zumindest 5 (fünf), bevorzugt zumindest 10 (zehn), weiter bevorzugt zumindest 50 (fünfzig) weitere bipolare Elektrodenpaare aufweist. Jedes dieser bipolaren Elektrodenpaare umfasst zwei Kleinkontaktelektroden mit einer Gewebekontaktfläche von jeweils kleiner 2 cm², und jede Elektrode ist mit einem Ausgang der Hochfrequenz-Energiequelle verbunden.

Wenn zuvor und im Folgenden davon die Rede ist, dass jede Elektrode eines Paares mit einem Ausgang der Hochfrequenz-Energiequelle verbunden ist, meint dies, dass jede Elektrode dieses einen Paares derart mit einem Ausgang der Hochfrequenz-Energiequelle verbunden ist, sodass zwischen den Elektroden (über das Gewebe) ein Strom fließen kann.

Solche oder ähnlich Elektrodenanordnungen sind aus der DE 10 2016 120 138 B4 und der EP 3 097 881 B1 bekannt, deren Offenbarung jeweils durch Bezugnahme vollständig mit in die vorliegende Beschreibung aufgenommen wird.

Speziell kann in Weiterbildung dieser Idee vorgesehen sein, dass eine erste Elektrode jedes Elektrodenpaares mit den ersten Elektroden der anderen Elektrodenpaare zu einer ersten Gesamtelektrode zusammengeschaltet ist und dass eine zweite Elektrode jedes Elektrodenpaares mit den zweiten Elektroden der anderen Elektrodenpaare zu einer zweiten Gesamtelektrode zusammengeschaltet ist, wobei ein aus der ersten Gesamtelektrode und der zweiten Gesamtelektrode gebildetes Elektrodenpaar mit einer zufälligen Abfolge von Frequenzen fⱼ beaufschlagt ist.

Diese Schaltungsanordnung ist speziell aus der DE 10 2016 120 138 B4 bekannt, und ihre Effizienz kann durch die vorgeschlagene zufällige Beaufschlagung der Elektroden noch verbessert werden.

Im Zuge einer anderen Weiterbildung dieser Idee kann noch vorgesehen sein, dass die Elektroden in einem bevorzugt regelmäßigen Muster aus Zeilen und Spalten angeordnet sind, wie dies ebenfalls aus der DE 10 2016 120 138 B4 bekannt ist. Vorzugsweise sind mit einem ersten Ausgang der Hochfrequenz-Energiequelle verbundene Elektroden in wenigstens einer ersten Zeile und mit einem zweiten Ausgang der Hochfrequenz-Energiequelle verbundene Elektroden in wenigstens einer zweiten Zeile angeordnet. Höchst vorzugsweise sind die ersten Zeilen und die zweiten Zeilen alternierend angeordnet.

Die Anmelderin hat erkannt, dass gerade bei einer solchen Elektrodenanordnung die vorgeschlagene zufällige Beaufschlagung gute Erfolge erzielen kann.

Weitere Eigenschaften und Vorteile der Erfindung ergeben sich aus der nachfolgenden Beschreibung von Ausführungsbeispielen anhand der Zeichnung.
Figur 1 zeigt eine erste Ausgestaltung der erfindungsgemäßen Vorrichtung;
Figur 2 zeigt eine zweite Ausgestaltung der erfindungsgemäßen Vorrichtung;
Figur 3 zeigt eine dritte Ausgestaltung der erfindungsgemäßen Vorrichtung;
Figur 4 zeigt eine erste Abfolge von Frequenzen zur Beaufschlagung von Elektroden einer erfindungsgemäßen Vorrichtung;
Figur 5 zeigt eine zweite Abfolge von Frequenzen zur Beaufschlagung von Elektroden einer erfindungsgemäßen Vorrichtung; und
Figur 6 zeigt eine dritte Abfolge von Frequenzen zur Beaufschlagung von Elektroden einer erfindungsgemäßen Vorrichtung.

Gleiche Bezugszeichen bezeichnen in allen Figuren gleiche oder zumindest gleichwirkende Elemente.

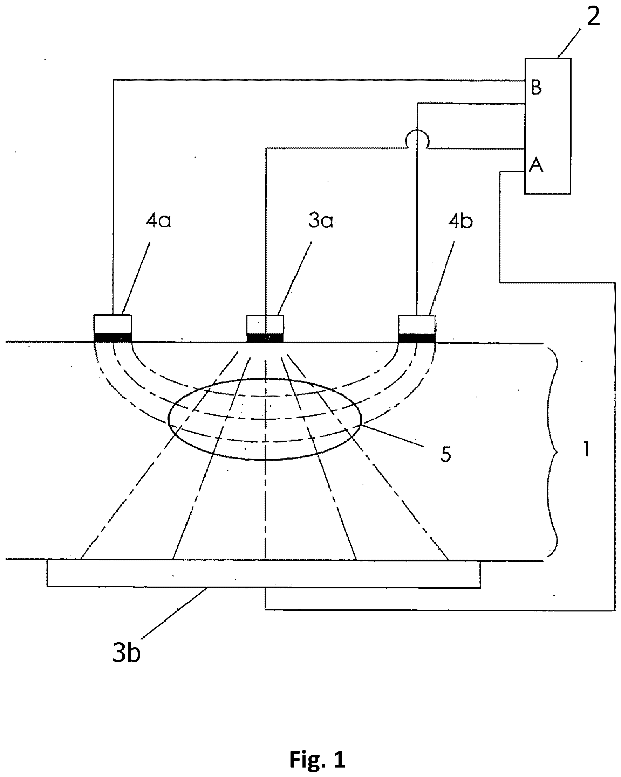

Die in Figur 1 dargestellte Vorrichtung dient zur kosmetischen und/oder therapeutischen Behandlung eines Gewebes 1. Hierzu werden mittels einer Hochfrequenz-Energiequelle 2 hochfrequente Ströme erzeugt, welche mittels Elektroden dem Gewebe 1 beaufschlagt werden.

Die Vorrichtung weist hierzu zwei Elektrodenpaare auf, von denen ein erstes Elektrodenpaar 3a, 3b als monopolares Elektrodenpaar ausgebildet ist und ein zweites Elektrodenpaar 4a, 4b als bipolares Elektrodenpaar ausgebildet ist. Die Vorrichtung in Figur 1 stellt somit eine tripolare Vorrichtung dar.

Entsprechend sind die Elektroden 3a, 4a und 4b als Kleinkontaktelektroden ausgeführt, jeweils mit einer Gewebekontaktfläche von etwa 5 mm². Die Elektrode 3b ist hingegen als Flächenkontaktelektrode ausgeführt und weist eine Gewebekontaktfläche von etwa 40 cm² auf.

Die Hochfrequenz (HF)-Energiequelle 2 weist zwei Ausgänge auf, wobei an einen ersten Ausgang A das erste Elektrodenpaar und an einen zweiten Ausgang B das zweite Elektrodenpaar angeschlossen ist. Mittels (nicht dargestellter) Wähltasten oder sonstiger Bedienelemente der HF-Energiequelle 2 kann für jedes Elektrodenpaar eine Frequenzbeaufschlagung vorgegeben werden. Insbesondere können eine minimale Frequenz von z.B. 0,1 MHz und eine maximale Frequenz von z.B. 20 MHz eingestellt werden. Außerdem kann eingestellt werden, mit welcher Leistung (Stromstärke) und mit welcher Pulslänge das Gewebe 1 durch die Elektroden des jeweiligen Ausgangs beaufschlagt wird. Dabei ist auch einstellbar, welches der Elektrodenpaare mit einer zufälligen Abfolge von Frequenzen beaufschlagt werden soll. Dies kann auch für beide Elektrodenpaare gelten, oder es kann eine alternierende Beaufschlagung der beiden Elektrodenpaare mit einer zufälligen Abfolge von Frequenzen ausgewählt werden. Für jede zufällige Abfolge von Frequenzen ist bevorzugt einstellbar, ob bzw. wie die Frequenzen (d.h. die entsprechenden Ströme) farbig gewichtet werden sollen. Sämtliche Parameter sind für jedes Elektrodenpaar separat vorgebbar, insbesondere können auch für beide Elektrodenpaare identische Parameter vorgegeben werden.

Besonders bevorzugt ist es, zumindest (auch) das monopolare Elektrodenpaar 3a, 3b stochastisch zu beaufschlagen, d.h. mit Strom zu beaufschlagen, dessen Frequenz zufällig variiert, vorzugsweise innerhalb der o.g. Grenzen.

Das Gewebe 1 ist in Figur 1 im Querschnitt dargestellt. Die gestrichelten Linien in Figur 1 geben Stromflusslinien an, entlang derer die Ströme zwischen den Elektroden 3a und 3b einerseits bzw. 4a und 4b andererseits fließen. In einem Überlagerungsbereich 5 findet eine Überlagerung der Ströme und damit eine erhöhte Erwärmung des Gewebes 1 statt.

Die Lage und insbesondere die Tiefe des Überlagerungsbereiches 5, das heißt der Abstand des Überlagerungsbereiches 5 von der in Figur 1 oberen Oberfläche des Gewebes 1 kann über den Abstand der Elektroden 4a und 4b zueinander, sowie über die Wahl der Frequenzen der hochfrequenten Ströme beeinflusst werden. Im Rahmen der vorliegenden Erfindung kann also auch ein zeitlich zufällig variierender Bereich behandelt werden.

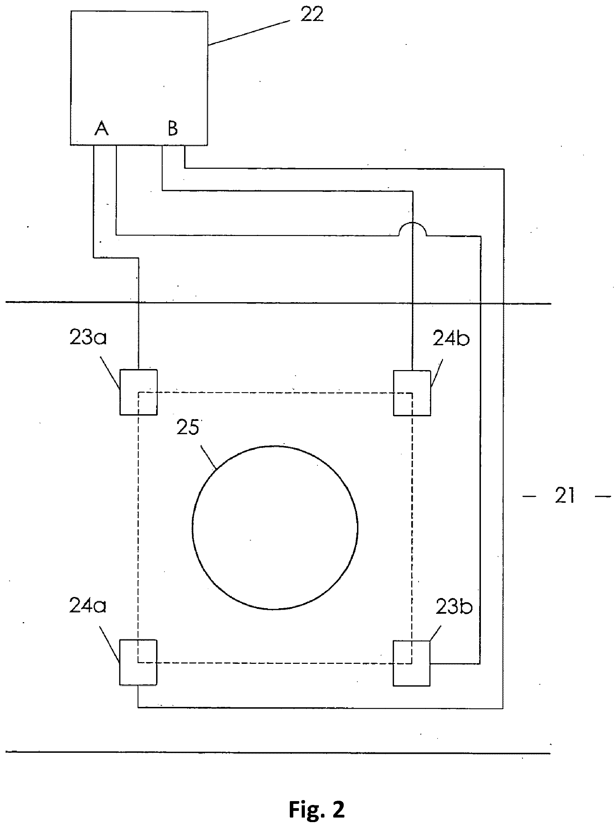

In Figur 2 ist eine erfindungsgemäße Vorrichtung mit zwei Elektrodenpaaren dargestellt, bei der beide Elektrodenpaare (23a und 23b, sowie 24a und 24b) jeweils als bipolares Elektrodenpaar ausgebildet sind. Die Vorrichtung in Figur 2 stellt somit insgesamt eine tetrapolare Vorrichtung dar.

Die Elektrodenpaare sind an Ausgänge A, B einer Hochfrequenz-Energiequelle 22 angeschlossen, welche analog zu der HF-Energiequelle 2 in Figur 1 ausgebildet ist und entsprechend betrieben werden kann.

Die Elektroden sind derart auf der Oberfläche eines Gewebes 21 angeordnet, dass sie auf den Eckpunkten eines (gestrichelt dargestellten) Quadrates liegen. Das Gewebe 21 ist somit in Figur 2 in Draufsicht dargestellt.

Dabei liegen sich die Elektroden eines bipolaren Elektrodenpaares jeweils auf dem Quadrat gegenüber. Hierdurch kann durch Wahl der Leistung und der Frequenz, mittels derer ein Elektrodenpaar durch die Energiequelle 22 beaufschlagt wird, die Wärmeentwicklung in einem Überlagerungsbereich 25 sowohl in der Intensität, das heißt in der Höhe der erzeugten Temperaturdifferenz, als auch in der Tiefe, das heißt dem Abstand zur Oberfläche des Gewebes 21 (in Figur 2 somit der Abstand in die Zeichenebene hinein) vorgegeben werden.

Beide Elektrodenpaare können zudem einzeln oder gemeinsam oder auch alternierend mit einer zufälligen Abfolge von Frequenzen (also mit Strömen, die eine solche Abfolge ihrer Frequenz aufweisen) beaufschlagt werden.

In der Praxis ist häufig die Verwendung von mehrpolaren Elektroden, insbesondere von mehrfachen bipolaren Elektroden wünschenswert.

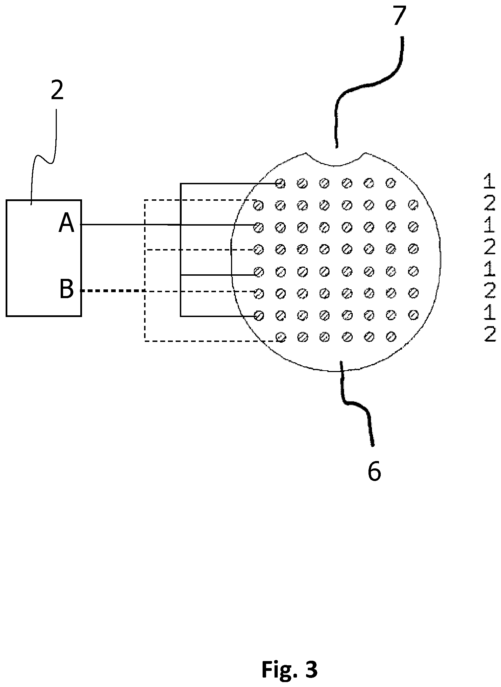

In Figur 3 ist beispielhaft ein (wechselbares) Elektrodenelement 6, wie es aus der DE 10 2016 120 138 B4 grundsätzlich bekannt ist, in Draufsicht auf die Behandlungsseite des Elektrodenelements dargestellt. Das Elektrodenelement 6 ist scheibenförmig ausgebildet und weist einen selektierenden Formbereich 7 auf. Hierdurch ist in Benutzungskonfiguration die exakte Orientierung des Elektrodenelements 6 in einem (hier nicht gezeigten) Basiselement gewährleistet.

Das Elektrodenelement 6 umfasst eine große Anzahl an bipolaren Elektroden. Dabei weist jede bipolare Elektrode weist zwei Teilelektroden auf. Eine erste Teilelektrode ist aus einer Zeile von jeweils punktartigen Kontaktflächen ausgebildet (in Figur 3 jeweils mit "1" markiert), welche untereinander elektrisch leitend verbunden sind. Eine dazugehörige zweite Teilelektrode ist ebenfalls aus einer Zeile von punktartigen Kontaktflächen ausgebildet (in Figur 2 jeweils mit "2" markiert), welche untereinander elektrisch leitend verbunden sind. Alle punktartigen Kontaktflächen einer Zeile sind also jeweils miteinander elektrisch leitend verbunden. Weiterhin sind alle Zeilen der Nummer 1 sowie alle Zeilen der Nummer 2 elektrisch miteinander verbunden und an jeweils einen (anderen) Ausgang A, B der HF-Energiequelle 2 angeschlossen, welche vorzugsweise analog zu der Energiequelle 2 in Figur 1 oder zu der Energiequelle 22 in Figur 2 ausgebildet ist und entsprechend betrieben werden kann. Die Zeilen "1" und "2" sind alternierend angeordnet.

Die Erfindung ist nicht auf die vorstehend beispielhaft gezeigten Elektrodenanordnungen beschränkt. Erfindungsgemäß ist es ausreichend, wenn wenigstens ein Elektrodenpaar vorhanden ist, das mit Strömen beaufschlagbar ist, deren Frequenzen eine zufällige Abfolge bilden.

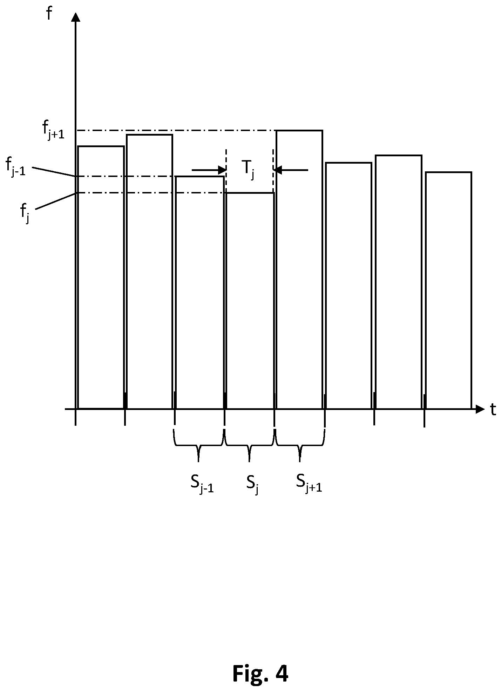

Figur 4 zeigt eine mögliche Abfolge von Frequenzen fⱼ (d.h. Strömen mit einer Frequenz fⱼ), die zur Beaufschlagung der in den Figuren 1 bis 3 beispielhaft erläuterten Elektrodenanordnungen oder zumindest Teilen davon genutzt werden kann.

Bezugszeichen t bezeichnet die Zeit(achse), während f die Frequenz(achse) angibt. Die Zeitachse ist in Sequenzschritte Sⱼ mit vorliegend konstanter Länge, d.h. in Zeitintervalle gleicher Dauer unterteilt. In jedem Sequenzschritt stellt die HF-Energiequelle (vgl. Figuren 1 bis 3) wenigstens eine Frequenz fⱼ bzw. einen (Wechsel-) Strom mit dieser Frequenz fⱼ während einer Zeitdauer Tⱼ zur Verfügung, der an wenigstens ein Elektrodenpaar der Vorrichtung angelegt ist.

Gemäß der Figur 4 sind alle Zeitdauern Tⱼ gleich lang, liegen unterhalb von 100 ms, vorzugsweise unterhalb 50 ms, weiter vorzugsweise unter 10 ms, und entsprechen (im Wesentlichen, also z.B. bis auf technisch bedingte kurze Pausen von etwa1 ms oder kürzer beim Umschalten der Frequenz) der Länge der einzelnen Sequenzschritte Sⱼ. Die Frequenzen fⱼ₋₁, fⱼ, fⱼ₊₁, ... folgen zufällig aufeinander und liegen zwischen 0,1 MHz und 20 MHz. Die zugehörigen Stromstärken liegen zwischen 100 mA und 2 A; sie können konstant sein oder zwischen den genannten Grenzen variieren.

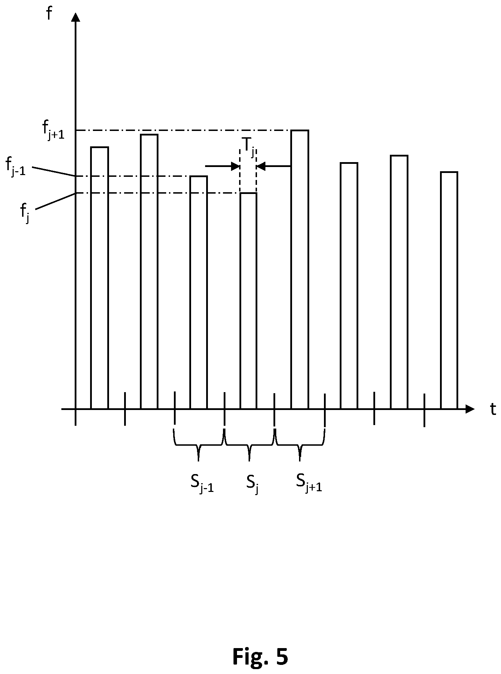

Figur 5 zeigt den abgewandelten Fall, bei dem die Zeitdauern Tⱼ zwar gleich lang sind (jeweils unterhalb von 100 ms, vorzugsweise unterhalb 50 ms, vorzugsweise unter 10ms), aber kürzer als die Länge der Sequenzschritte Sⱼ. Exemplarisch und ohne Beschränkung erfolgt die Beaufschlagung immer genau in der Mitte jedes Sequenzschritts Sⱼ. Die Frequenzen fⱼ₋₁, fⱼ, fⱼ₊₁, ... folgen wiederum zufällig aufeinander und liegen zwischen 0,1 MHz und 20 MHz. Die zugehörigen Stromstärken liegen wiederum zwischen 100 mA und 2 A; sie können konstant sein oder zwischen den genannten Grenzen variieren.

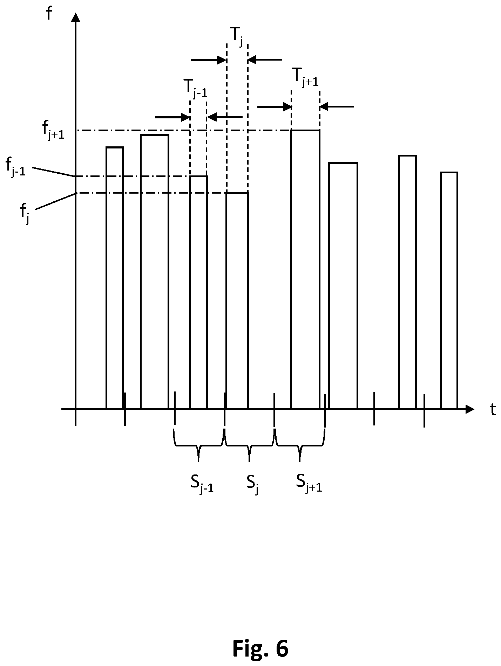

Figur 6 zeigt einen abermals abgewandelten Fall, bei dem weder die Zeitdauern Tⱼ gleich lang sind (jeweils unterhalb von 100 ms, vorzugsweise unterhalb 50 ms), noch die Beaufschlagung immer genau in der Mitte jedes Sequenzschritts Sⱼ erfolgt. Vielmehr sind vorliegend auch die Zeitdauern Tⱼ₋₁, Tⱼ, Tⱼ₊₁, ... zufällig gewählt (und zwar nach ihrer jeweiligen Dauer und Lage innerhalb des zugehörigen Sequenzschritts Sⱼ, wobei hier grundsätzlich einer der beiden Parameter ausreichend ist). Die Frequenzen fⱼ₋₁, fⱼ, fⱼ₊₁, ... folgen erneut zufällig aufeinander und liegen zwischen 0,1 MHz und 20 MHz. Die zugehörigen Stromstärken liegen zwischen 100 mA und 2 A; sie können konstant sein oder zwischen den genannten Grenzen variieren.

In Abwandlung der Lehre der Figur 6 kann auch vorgesehen sein, wie einleitend beschrieben, dass die einzelnen Sequenzschritte Sⱼ₋₁, Sⱼ, Sⱼ₊₁, ... jeweils unterschiedlich lang gewählt sind und dass die Zeitdauern Tⱼ₋₁, Tⱼ, Tⱼ₊₁, ... wiederum den Sequenzschritten bezüglich ihrer jeweiligen Dauer (bis auf kurze technisch bedingte Umschaltzeiten von ca. 1 ms oder kürzer) entsprechen, wie dies in Figur 4 gezeigt wurde.

Bei einem konkreten Anwendungsbeispiel werden nach diesem letztgenannte Schema 45 Pulse (ein sog. Pulsetrain oder "long pulse") mit stochastisch gewählten Frequenzen zwischen 0,1 MHz und ca. 7 MHz erzeugt, die eine Pulsbreite (Zeitdauer) zwischen 10 ms und 30 ms und eine Stromstärke zwischen 200 mA und 500 mA aufweisen. Analog zu weißem Rauschen sind dabei die Frequenzen gleichmäßig zwischen den genannten Grenzen verteilt angeordnet.

Ein "Pulsetrain" bzw. "long pulse"umfasst demnach eine bestimmte Anzahl von Pulsen, dann endet die Behandlung bevorzugt automatisch. Es gibt üblicherweise einen Knopf, z.B. an einem Behandlungs-Handstück, das von einer behandelnden Person geführt wird: auf Knopfdruck - typischerweise nach Bewegen des Handstücks an einen anderen Ort - folgt dann der nächste Pulsetrain oder "long pulse".

## Patentansprüche

1. Vorrichtung zur Erzeugung von hochfrequenten Strömen, zur kosmetischen und/oder therapeutischen Behandlung von Gewebe (1, 21) mittels Elektroden (3a, 3b; 4a, 4b; 23a, 23b; 24a, 24b) der Vorrichtung, umfassend:
eine Hochfrequenz-Energiequelle (2, 22) zur Erzeugung von hochfrequenten Strömen und mindestens ein erstes Elektrodenpaar mit zwei Elektroden (3a, 3b; 4a, 4b; 23a,
23b; 24a, 24b) zum Beaufschlagen des Gewebes (1, 21) mit den hochfrequenten Strömen, welche Elektroden (3a, 3b; 4a, 4b; 23a, 23b; 24a, 24b) mit jeweils einem Ausgang (A, B) der Hochfrequenz-Energiequelle (2, 22) verbunden sind,
welche Vorrichtung ausgebildet ist, mittels der Hochfrequenz-Energiequelle (2, 22) die hochfrequenten Ströme mit mehreren unterschiedlichen Frequenzen fⱼ zu erzeugen,
**dadurch gekennzeichnet, dass**
die Vorrichtung ausgebildet ist, mittels der Hochfrequenz-Energiequelle (2, 22) die Ströme mit mehreren unterschiedlichen Frequenzen fⱼ in einer zeitlichen Sequenz mit Sequenzschritten Sⱼ mit j=1, 2, ..., M; M E N, zu erzeugen,
jedem Sequenzschritt Sⱼ zumindest ein in diesem Sequenzschritt Sⱼ erzeugter Strom mit der Frequenz fⱼ und eine Zeitdauer Tⱼ, für welche der Strom mit der Frequenz fⱼ in dem Sequenzschritt Sⱼ erzeugt wird, zugeordnet ist, und
zumindest eine Abfolge der Frequenzen fⱼ eine zufällige Abfolge ist, und dass die Vorrichtung zusätzlich ausgebildet ist,
die Ströme mit einer Stromstärke zwischen 100 mA und 2 A, vorzugsweise zwischen 300 mA und 1 A zu erzeugen, und/oder
die Ströme in einem Frequenzbereich zwischen 0,1 MHz und 20 MHz, vorzugsweise zwischen 0,1 MHz und 10 MHz, höchst vorzugsweise zwischen 0,1 MHz und 3 MHz, zu erzeugen, und/oder
die Ströme während einer Zeitdauer Tⱼ, kleiner 100 ms, vorzugsweise kleiner 50 ms, zu erzeugen.

2. Vorrichtung nach Anspruch 1,
**dadurch gekennzeichnet, dass**
zusätzlich eine Abfolge der Zeitdauern Tⱼ, eine zufällige Abfolge ist.

3. Vorrichtung nach Anspruch 1 oder 2,
**dadurch gekennzeichnet, dass**
die Vorrichtung ausgebildet ist, mittels der Hochfrequenz-Energiequelle (2, 22) die Ströme mit mehreren unterschiedlichen Frequenzen fⱼ zu erzeugen, sodass die Frequenzen fⱼ gleichmäßig oder gemäß einer vorgegebenen Gewichtungsfunktion g(f) über den Frequenzbereich verteilt sind.

4. Vorrichtung nach einem der Ansprüche 1 bis 3,
**dadurch gekennzeichnet, dass**
die Vorrichtung ausgebildet ist zum Erzeugen von langen Pulsen mit einer Dauer von mindestens 1 s, vorzugsweise 5 s, höchst vorzugsweise 10 s, während der das Gewebe an einem festen Ort mit den hochfrequenten Strömen beaufschlagt ist.

5. Vorrichtung nach einem der Ansprüche 1 bis 4,
**dadurch gekennzeichnet, dass**
die Vorrichtung zumindest ein zweites Elektrodenpaar mit zwei Elektroden (3a, 3b; 4a, 4b; 23a, 23b; 24a, 24b) aufweist, die mit jeweils einem Ausgang (A, B) der Hochfrequenz-Energiequelle (2, 22) verbunden sind, und
die Hochfrequenz-Energiequelle (2, 22) ausgebildet ist, die zwei Elektrodenpaare in wenigstens einem Sequenzschritt Sⱼ, vorzugsweise in im Wesentlichen allen Sequenzschritten Sⱼ, mit Strömen unterschiedlicher Frequenz fᵢ zu beaufschlagen.

6. Vorrichtung nach Anspruch 5,
**dadurch gekennzeichnet, dass**
eines der Elektrodenpaare als monopolares Elektrodenpaar ausgebildet ist, das eine Flächenkontaktelektrode (3b) mit einer Gewebekontaktfläche größer 5 cm² und eine Kleinkontaktelektrode (3a) mit einer Gewebekontaktfläche kleiner 2 cm² umfasst, und
das andere Elektrodenpaar als bipolares Elektrodenpaar ausgebildet ist, das zwei Kleinkontaktelektroden (4a, 4b; 23a, 23b; 24a, 24b) mit einer Gewebekontaktfläche von jeweils kleiner 2 cm² umfasst.

7. Vorrichtung nach Anspruch 5 oder 6,
**dadurch gekennzeichnet, dass**
eines der Elektrodenpaare mit Strömen mit einer zufälligen Abfolge von Frequenzen fⱼ beaufschlagt ist, vorzugsweise das monopolare Elektrodenpaar (3a, 3b), und das andere Elektrodenpaar (4a, 4b) mit Strömen mit einer vorbestimmten Abfolge von Frequenzen fⱼ beaufschlagt ist.

8. Vorrichtung nach Anspruch 5 oder 6,
**dadurch gekennzeichnet, dass**
beide Elektrodenpaare mit Strömen mit einer zufälligen Abfolge von Frequenzen fⱼ beaufschlagt sind.

9. Vorrichtung nach Anspruch 5 oder 6,
**dadurch gekennzeichnet, dass**
beide Elektrodenpaare alternierend mit Strömen mit einer zufälligen Abfolge von Frequenzen fⱼ beaufschlagt sind.

10. Vorrichtung nach einem der Ansprüche 1 bis 4,
**dadurch gekennzeichnet, dass**
das erste Elektrodenpaar als bipolares Elektrodenpaar (23a, 23b) ausgebildet ist, und die Vorrichtung eine Mehrzahl weiterer bipolare Elektrodenpaare (24a, 24b), insbesondere zumindest 5, bevorzugt zumindest 10, weiter bevorzugt zumindest 50 weitere bipolare Elektrodenpaare aufweist,
von denen jedes bipolare Elektrodenpaar zwei Kleinkontaktelektroden (23b, 23b; 24a, 24b) mit einer Gewebekontaktfläche von jeweils kleiner 2 cm² umfasst und jede Elektrode mit einem Ausgang (A, B) der Hochfrequenz-Energiequelle (2, 22) verbunden ist.

11. Vorrichtung nach Anspruch 10,
**dadurch gekennzeichnet, dass**
eine erste Elektrode jedes Elektrodenpaares mit den ersten Elektroden der anderen Elektrodenpaare zu einer ersten Gesamtelektrode zusammengeschaltet ist und eine zweite Elektrode jedes Elektrodenpaares mit den zweiten Elektroden der anderen Elektrodenpaare zu einer zweiten Gesamtelektrode zusammengeschaltet ist, wobei ein aus der ersten Gesamtelektrode und der zweiten Gesamtelektrode gebildetes Elektrodenpaar mit einer zufälligen Abfolge von Frequenzen fⱼ beaufschlagt ist.

12. Vorrichtung nach Anspruch 10 oder 11,
**dadurch gekennzeichnet, dass**
die Elektroden in einem bevorzugt regelmäßigen Muster aus Zeilen und Spalten angeordnet sind, wobei vorzugsweise mit einem ersten Ausgang (A) der Hochfrequenz-Energiequelle (2) verbundene Elektroden in wenigstens einer ersten Zeile und mit einem zweiten Ausgang (B) der Hochfrequenz-Energiequelle (2) verbundene Elektroden in wenigstens einer zweiten Zeile angeordnet sind, wobei höchst vorzugsweise die ersten Zeilen und die zweiten Zeilen alternierend angeordnet sind.

13. Verfahren zur Behandlung von Gewebe (1, 21), insbesondere zur kosmetischen Behandlung von Gewebe (1, 21), mittels hochfrequenten Strömen,
mit den Verfahrensschritten
- Anordnen zumindest eines Elektrodenpaars (3a, 3b; 4a, 4b; 23a, 23b; 24a, 24b) an dem Gewebe (1, 21),
- Beaufschlagen des Gewebes (1, 21) mittels des Elektrodenpaares (3a, 3b; 4a, 4b; 23a, 23b; 24a, 24b) mit hochfrequentem Strom,
**dadurch gekennzeichnet, dass**
das Gewebe (1, 21) sequentiell mit Strömen mit mehreren unterschiedlichen Frequenzen fⱼ beaufschlagt wird, die in einer zeitlichen Sequenz mit Sequenzschritten Sⱼ mit j=1, 2, ..., M; M E N, aufeinander folgen,
wobei in jedem Sequenzschritt Sⱼ das Gewebe mit Strom einer diesem Sequenzschritt zugeordneten Frequenz fⱼ während einer Zeitdauer Tⱼ, beaufschlagt wird, und zumindest die Frequenzen fⱼ zufällig aufeinander folgen,
während zusätzlich wenigstens eines der folgenden weiteren Merkmale realisiert wird:
eine Stromstärke der Ströme wird zwischen 100 mA und 2 A, vorzugsweise zwischen 300 mA und 1 A, gewählt; und/oder
das Gewebe (1, 21) wird mit Strömen mit einer Frequenz fⱼ in einem Frequenzbereich zwischen 0,1 MHz und 20 MHz, vorzugsweise zwischen 0,1 MHz und 10 MHz, höchst vorzugsweise zwischen 0,1 MHz und 3 MHz, beaufschlagt; und/oder die Zeitdauer Tⱼ, beträgt weniger als 100 ms, vorzugsweise weniger als 50 ms.

14. Verfahren nach Anspruch 13,
**dadurch gekennzeichnet, dass**
zusätzlich die Zeitdauern Tⱼ, zufällig aufeinander folgen.

15. Verfahren nach Anspruch 13 oder 14,
**dadurch gekennzeichnet, dass**
die Ströme mit mehreren unterschiedlichen Frequenzen fⱼ erzeugt werden, die gleichmäßig oder gemäß einer vorgegebenen Gewichtungsfunktion g(f) über den Frequenzbereich verteilt sind.

16. Verfahren nach einem der Ansprüche 13 bis 15,
**dadurch gekennzeichnet, dass**
das Elektrodenpaar (3a, 3b; 4a, 4b; 23a, 23b; 24a, 24b) für eine Dauer von mindestens 1 s, vorzugsweise 5 s, höchst vorzugsweise 10 s, während der das Gewebe mit den hochfrequenten Strömen beaufschlagt wird, an einem festen Ort an dem Gewebe (1, 21) angeordnet wird.
